(19) Europäisches Patentamt
European Patent Office
Office européen des brevets

(11) **EP 4 085 837 A1**

(12) **EUROPEAN PATENT APPLICATION**

(43) Date of publication:
**09.11.2022 Bulletin 2022/45**

(21) Application number: **21172335.8**

(22) Date of filing: **05.05.2021**

(51) International Patent Classification (IPC):
*A61B 5/145* (2006.01)

(52) Cooperative Patent Classification (CPC):
**A61B 5/14517; A61B 5/14546; A61B 5/6826;
A61B 5/6843**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **VAN LIESHOUT, Ron Martinus Laurentius
5656 AE Eindhoven (NL)**

• **DELLIMORE, Kiran Hamilton J.
5656 AE Eindhoven (NL)**
• **PELSSERS, Eduard Gerard Marie
5656 AE Eindhoven (NL)**
• **JOHNSON, Mark Thomas
5656 AE Eindhoven (NL)**

(74) Representative: **Philips Intellectual Property &
Standards
High Tech Campus 52
5656 AG Eindhoven (NL)**

(54) **DETERMINING THE CONCENTRATION OF A BIOMARKER IN BLOOD OF A SUBJECT**

(57) According to an aspect, there is provided a method (200, 400) for measuring a concentration of a biomarker in sweat generated by a subject, the method comprising: determining (202) a first concentration of the biomarker in sweat secreted from a sweat gland of the subject; applying (204) an external positive pressure to the subject at the location of the sweat gland; measuring (206), while the external positive pressure is being applied, a second concentration of the biomarker in sweat secreted from the sweat gland of the subject; and calculating (208), based on the determined first concentration and the measured second concentration, a third concentration of the biomarker, the third concentration relating to the concentration of the biomarker in the blood of the subject.

200

Fig. 2

EP 4 085 837 A1

**Description**

FIELD OF THE INVENTION

The invention relates to determining the concentration of a biomarker in blood of a subject and, more particularly, to determining the concentration of a biomarker in a subject's blood based on a measurement of the concentration of the biomarker in sweat of the subject.

BACKGROUND OF THE INVENTION

Non-invasive, continuous and prolonged monitoring of biomarkers that indicate health and well-being is useful for example for monitoring dehydration, stress, sleep, children's health and for perioperative monitoring. Sweat is a non-obtrusively accessible bio-fluid containing physiologically and metabolically rich information. Some examples of clinically relevant components of sweat are sodium ions, chlorine ions and potassium ions to monitor dehydration, lactate as an early warning for inflammation (relevant to sepsis), glucose for diabetes and neonates and cortisol for sleep and stress monitoring. Therefore, analysing components in sweat can provide useful medical information about a person.

However, the correlation between concentrations of components in blood and sweat is lacking for some biomarkers. In other words, the concentration of a biomarker in sweat of a person may not accurately represent the concentration of the biomarker in the person's blood.

SUMMARY OF THE INVENTION

There is a desire to be able to take account of the difference between the concentration of a component (such as a biomarker) in sweat and its concentration in blood, and take action to mitigate the difference, such that a measurement of the component's concentration in sweat can be taken as an accurate representation of the component's concentration in blood. The invention is defined by the independent claims. The dependent claims define advantageous embodiments.

The inventors have recognised that various mechanisms that occur within the human body affect the concentration of biomarkers measurable in sweat. One such mechanism involves the movement of fluids into and out of capillary beds into interstitial fluid and cells surrounding blood capillaries, sometimes referred to as capillary exchange. According to various embodiments disclosed herein, measurements of the concentration of a biomarker are taken under normal conditions, and again when external positive pressure is being applied to the person. As used herein, the term "positive pressure" is intended to refer to a pressure that is greater than (e.g. exceeds) air pressure (e.g. the ambient air pressure). For example, a positive pressure may be applied by exerting a force on a surface. From the two measurements, it is possible to determine a concentration of the biomarker in the sweat that corresponds to the concentration of the biomarker in the person's blood.

According to a first specific aspect, there is provided a method for measuring a concentration of a biomarker in sweat generated by a subject, the method comprising: determining a first concentration of the biomarker in sweat secreted from a sweat gland of the subject; applying an external positive pressure to the subject at the location of the sweat gland; measuring, while the external positive pressure is being applied, a second concentration of the biomarker in sweat secreted from the sweat gland of the subject; and calculating, based on the determined first concentration and the measured second concentration, a third concentration of the biomarker, the third concentration relating to the concentration of the biomarker in the blood of the subject.

In some embodiments, the method may further comprise measuring, prior to applying the external positive pressure to the subject, a first sweat rate of the subject. The method may further comprise measuring, after expiry of a defined duration, a second sweat rate of the subject; wherein calculating the third concentration of the biomarker is further based on the measured first sweat rate and the measured second sweat rate.

In some embodiments, applying an external pressure may comprise exerting a force onto skin of the subject at the location of the sweat gland, so as to increase a pressure in interstitial fluid around the sweat gland. In another embodiment, the method may further comprise applying, after measuring the second concentration of the biomarker, a negative pressure to the subject at the location of the sweat gland.

The method may, in some embodiments, further comprise measuring, during the application of the external positive pressure, a plurality of concentrations of the biomarker. Calculating the third concentration may be further based on the plurality of measurements.

In some embodiments, the method may further comprise measuring a blood pressure of the subject. Applying the external positive pressure may comprise applying a positive pressure to equalise a pressure of interstitial fluid around the sweat gland with the measured blood pressure.

Applying the external positive pressure may comprise, after measuring the second concentration of the biomarker, decreasing the applied pressure in regular steps, and measuring, at each step, a concentration of the biomarker in sweat secreted from the sweat gland of the subject. The method may further comprise generating a calibration curve using the measured concentrations. Calculating the third concentration of the biomarker may be further based on the generated calibration curve.

According to a second specific aspect, there is provided an apparatus for measuring a concentration of a biomarker in sweat generated by a subject, the apparatus comprising: a biomarker sensor for receiving a volume of sweat secreted from a sweat gland of the subject and measuring a concentration of a biomarker in the volume of sweat; a pressure application unit configured to apply a positive pressure to the subject at the location of the sweat gland; and a processor in communication with the biomarker sensor and the pressure application unit, and configured to: determine a first concentration of the biomarker in a first volume of sweat secreted from the sweat gland while the pressure application unit is not applying a pressure to the subject; operate the pressure application unit to apply a positive pressure to the subject at the location of the sweat gland; operate the biomarker sensor to measure a second concentration of the biomarker in a second volume of sweat secreted from the sweat gland while the pressure application unit is applying a positive pressure to the subject; and calculate, based on the determined first concentration and the measured second concentration, a third concentration of the biomarker, the third concentration relating to the concentration of the biomarker in the blood of the subject.

The apparatus may further comprise a blood pressure measurement unit for measuring a blood pressure of the subject. The processor may be configured to operate the pressure application unit to apply a positive pressure based on the measured blood pressure of the subject.

In some embodiments, the blood pressure measurement unit may be configured to measure a capillary hydrostatic pressure of the subject. The processor may be configured to operate the pressure application unit to apply a positive pressure to the subject such that a hydrostatic pressure in interstitial fluid around the sweat gland is increased to equal the measured capillary hydrostatic pressure.

The apparatus may, in some embodiments, further comprise a timer in communication with the processor, and configured to measure at least one of: a duration of application of the applied pressure; and the timing of measurements of the biomarker concentrations.

In some embodiments, the apparatus may further comprise a sweat rate measurement unit configured to measure a sweat rate of the subject. The processor may be configured to control the sweat rate measurement unit to measure a first sweat rate of the subject prior to applying the external positive pressure to the subject; control the sweat rate measurement unit to measure a second sweat rate of the subject after expiry of a defined duration; and calculate the third concentration of the biomarker further based on the measured first sweat rate and the measured second sweat rate.

The processor may be configured to operate the biomarker sensor to measure a plurality of concentrations of the biomarker in volumes of sweat secreted from the sweat gland while the pressure application unit is applying a positive pressure to the subject; and calculate the third concentration further based on the plurality of biomarker concentration measurements.

In some embodiments, the biomarker sensor may be configured to measure the concentration, in the volume of sweat, of a biomarker selected from a group comprising: lactate, sodium ions, potassium ions, chlorine ions, urea, ethanol, ammonium, glucose and cortisol.

According to a third specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to carry out any of the above methods for measuring a concentration of a biomarker in sweat generated by a subject. These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a schematic illustration of an example of an apparatus in accordance with various embodiments;
Fig. 2 is a flowchart of an example of a method of measuring a concentration of a biomarker in sweat generated by a subject;
Fig. 3 is a flowchart of a further example of a method of measuring a concentration of a biomarker in sweat generated by a subject;
Fig. 4 is a flowchart of a further example of a method of measuring a concentration of a biomarker in sweat generated by a subject;
Fig. 5 is a schematic illustration of an example of a technique for applying a negative pressure;

Fig. 6 is a schematic illustration of a further example of a technique for applying a negative pressure;

Fig. 7 shows two graphs relating to generating calibration data;

Fig. 8 is a schematic illustration of a further example of an apparatus in accordance with various embodiments; and

Fig. 9 is a schematic illustration of an example of a processor in communication with a computer-readable medium.

## DETAILED DESCRIPTION OF EMBODIMENTS

Embodiments disclosed herein provide a mechanism by which a measurement of the concentration of a biomarker in a subject's sweat can be correlated with the concentration of the biomarker in the subject's blood, and this is achieved by measuring the concentration of the biomarker in the sweat both under normal conditions (e.g. without an external pressure being applied to the subject) and while an external pressure (i.e. a positive pressure) is being applied to the subject. Applying an external positive pressure to the skin of the subject in the vicinity of the sweat glands from which the subject sweat is generated helps to counteract various effects that take place within the body that affect the concentration of certain biomarkers in the subject's sweat. In this disclosure, unless stated otherwise, reference to a "pressure" being applied to a surface is intended to refer to a positive pressure, caused for example by the exertion of a force onto the surface, such as the skin of a subject. Such a positive pressure may cause an increased pressure in tissue under the surface where the pressure is applied.

Capillary exchange refers to the mass movement of fluids into and out of capillary beds (i.e. the blood capillary system of a person) and involves two pressure-driven mechanisms. A first of the mechanisms occurs when fluid moves from an area of relatively higher pressure in a capillary bed to an area of relatively lower pressure in tissue next to, or adjacent to, the capillary bed via a process known as filtration. A second mechanism occurs when fluid moves from an area of relatively higher pressure in the tissue to an area of relatively lower pressure in the capillary bed via a process known as reabsorption. Two types of pressure interact to drive these movements: hydrostatic pressure and osmotic pressure.

Hydrostatic pressure is the main contributor of fluid transport between the capillaries and tissue, and can be considered to represent the pressure of a fluid enclosed in a space. Blood hydrostatic pressure is caused by blood confined within blood vessels or heart chambers. More specifically, the pressure exerted by blood against the wall of a capillary is referred to as capillary hydrostatic pressure (CHP), or capillary blood pressure. Tissue surrounding capillaries includes interstitial fluid. As fluid exits a capillary and moves into the surrounding tissue, the hydrostatic pressure in the interstitial fluid increases correspondingly, and is referred to as the interstitial fluid hydrostatic pressure (IFHP). Generally, the CHP originating from arterial pathways is considerably higher than the IFHP, because lymphatic vessels act to absorb excess fluid from the tissues. Thus, fluid generally tends to move out of the capillaries and into the interstitial fluid in the process referred to as filtration.

Osmotic pressure (sometimes referred to as oncotic pressure) acts against the hydrostatic pressure, and serves to draw fluid from the interstitial fluid back into the capillaries.

Filtration out of, and reabsorption into, a capillary occur in accordance with the balance of hydrostatic pressure and osmotic pressure, as described in the Starling hypothesis:

$$Filtration\ force = k[(P_c + \pi_i) - (P_i + \pi_c)] \qquad [1]$$

where $k$ is a permeability coefficient; $P_c$ = hydrostatic (blood) pressure in the capillary; $\pi_i$ = oncotic pressure in the interstitial fluid; $P_i$ = hydrostatic pressure in the interstitial fluid; and $\pi_c$ = oncotic pressure in the capillary (e.g. from plasma proteins). $P_c$ and $P_i$ are "positive pressures" and $\pi_i$ and $\pi_c$ are "negative pressures" (generated by osmosis). Biomolecules, including those which may be detected and used as biomarkers, may pass with fluid from the capillary into the interstitial fluid during the filtration process.

Other processes taking place within the body also contribute to the movement of fluid containing biomarkers. Biomarkers present in sweat within the body can be reabsorbed into capillaries if, for example, the hydrostatic pressure in the interstitial fluid is greater than the hydrostatic pressure in the capillary. Furthermore, biomarkers may be caused to move into the interstitial fluid by the process of perfusion around the sweat gland. Perfusion is a process by which fluid (e.g. fluid containing biomarkers) moves up through a circulatory system to an organ (e.g. skin) or tissue. Movement of fluids through a subject's body can be affected or influenced by the increase of pressure in the subject's tissue and interstitial fluid (e.g. by the application of external pressure on the subject's skin).

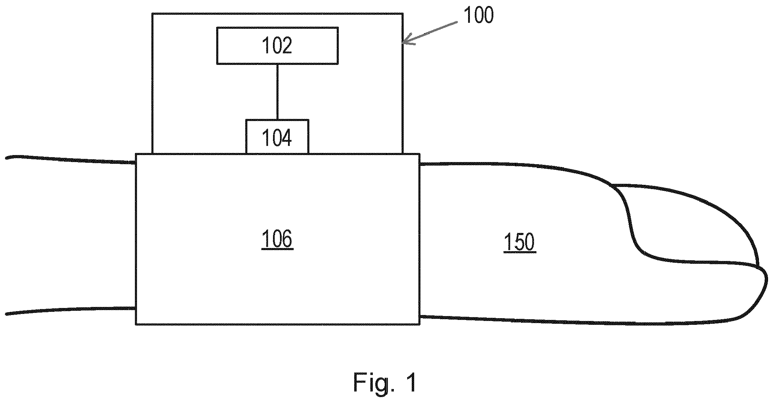

Referring to the drawings, Fig. 1 is a schematic illustration of an example of an apparatus 100 for measuring a concentration of a biomarker in sweat generated by a subject. In this example, the apparatus is shown attached to a finger 150 of the subject. However, in other embodiments, the apparatus 100 may be used with, and attached to, other parts of the subject's body.

The apparatus 100 comprises a processor 102, a biomarker sensor 104 and a pressure application unit 106.

The biomarker sensor 104 is for receiving a volume of sweat secreted from a sweat gland of the subject and measuring a concentration of a biomarker in the volume of sweat. In use, the biomarker sensor 104 may be positioned such that it contacts the skin of the subject (e.g. skin of the finger 150) to receive from pores in the skin. In other examples, sweat may be transported from the skin to the biomarker sensor 104 via a fluid transport mechanism, such as a microfluidic system. The biomarker sensor 104 may contain components (not shown) configured to detect one or more specific biomarkers (e.g. biomolecules having a particular characteristic and/or indicative of a particular condition) present in the volume of sweat.

The pressure application unit 106 is configured to apply a pressure to the subject at the location of the sweat gland. As discussed in greater detail below, the pressure applied using the pressure application unit 106 may comprise a positive pressure or over-pressure (i.e. intended to increase the pressure in tissue surrounding the sweat gland). In some examples, the pressure application unit 106 or another unit may be configured to apply a negative pressure or under-pressure (i.e. intended to reduce the pressure in tissue surrounding the sweat gland), as is discussed below. As used herein, a "negative" pressure is intended to refer to a pressure below ambient air pressure. A negative pressure may be referred to as an under-pressure, and/or may be said to be vacuum assisted. In some examples, a negative pressure may be created by using a suction pump or vacuum pump, for example by reducing the pressure in the region above the subject's skin, as described herein. In the example shown, the pressure application unit 106 comprises a cuff that wraps around the finger 150 and is inflatable to apply a positive pressure to the finger at the location of the sweat gland. It will be understood that the sweat gland is located under the subject's skin and that, by applying pressure to the finger 150 pressure is also applied to tissue under the skin and, accordingly, to tissue surrounding the sweat gland. In embodiments in which the apparatus 100 is to be used with a different part of the subject's body, the pressure application unit 106 may be formed differently. For example, the pressure application unit 106 may comprise an arm cuff configured to be attached around an arm of the subject, or a cuff forming part of a wearable device such as a smart watch, configured to be attached around the wrist of the subject.

The processor 102 is in communication with the biomarker sensor 104 and the pressure application unit 106. The processor 102 is configured to determine a first concentration of the biomarker in a first volume of sweat secreted from the sweat gland while the pressure application unit 106 is not applying a pressure (i.e. a positive pressure) to the subject. In some embodiments, the processor 102 may determine the first concentration by obtaining the first concentration from a database or a lookup table. For example, such a database or a lookup table may include data indicative of the concentrations of various biomarkers in sweat under standard pressure (e.g. when no external positive pressure is applied). In other embodiments, the processor 102 may determine the first concentration through measurement. For example, the processor 102 may be configured to operate the biomarker sensor 104 to measure the first concentration. In this example, the biomarker sensor 104 measures a first concentration of the biomarker in sweat secreted from the sweat gland under normal conditions, without external pressure being applied to the skin of the subject by the pressure application unit 106. The biomarker measured using the biomarker sensor 104, or whose concentration is obtained by the processor 102, may comprise any biomarker appropriate for determining information about the sweat and may, for example, comprise any biomarker discussed herein. In one specific example, the biomarker sensor 104 may be configured to measure a concentration of lactate in the sweat.

By determining (e.g. measuring or otherwise obtaining) the concentration of the biomarker in the sweat when no external positive pressure is being applied to the subject in the vicinity of the sweat glands secreting the sweat, the determined biomarker concentration includes contributions from the sweat glands themselves (e.g. a concentration of the biomarker in sweat generated by the sweat glands) and from components in the blood moving into the interstitial fluid and into the sweat gland from the capillaries by capillary exchange and/or filtration (e.g. a concentration of the biomarker in sweat, originating from the capillaries), which is sometimes referred to diffusion between blood and the sweat gland via the interstitial fluid.

The processor 102 is further configured to operate the pressure application unit 106 to apply a positive pressure to the subject at the location of the sweat gland. In some embodiments, the processor 102 may be configured to operate the pressure application unit 106 for a defined duration. In some embodiments, a positive pressure may be applied in order to increase an effective hydrostatic pressure in the interstitial fluid (i.e. increasing $P_i$ in equation [1]). The amount of external positive pressure to be applied to the subject using the pressure application unit 106 may depend on the subject (e.g. on a blood pressure of the subject). In general, the pressure application unit 106 may apply a pressure that is equal to or greater than the hydrostatic blood pressure ($P_c$), in order to reduce transport of the fluid and biomarker from the capillary bed to the interstitial fluid by filtration. In some embodiments, the pressure application unit 106 may apply a pressure approximately equal to, or greater than, 35 mmHg.

The processor 102 is further configured to operate the biomarker sensor 104 to measure a second concentration of the biomarker in a second volume of sweat secreted from the sweat gland while the pressure application unit 106 is applying a positive pressure to the subject. In other words, during the application of pressure, the biomarker sensor 104 again measures the concentration of the biomarker in sweat secreted from the sweat gland. While the positive pressure is being applied to the subject by the pressure application unit 106, the hydrostatic pressure of the interstitial fluid is increased, transport of biomarker-containing fluid from the capillaries to the interstitial fluid is reduced and, therefore, the main source of the biomarker in sweat is the biomarker originating from the sweat glands themselves.

The processor 102 is further configured to calculate, based on the determined first concentration and the measured second concentration, a third concentration of the biomarker, the third concentration relating to the concentration of the biomarker in the blood of the subject. In the example, the measured second concentration (i.e. the concentration of the biomarker originating from the sweat glands) may be subtracted from the determined first concentration (i.e. the concentration of the biomarker originating from the sweat glands and from capillaries) to yield the third concentration, which is representative of the concentration of the biomarker from the capillary bed.

While, in the embodiment shown in Fig. 1, the processor 102 forms part of the apparatus 100 along with the biomarker sensor 104 and the pressure application unit 106, it will be appreciated that, in other embodiments, the processor 102 may be located remotely with respect to the biomarker sensor and/or the pressure application unit. For example, separate apparatuses may each include one of the biomarker sensor 104 and the pressure application unit 106, and these may each communicate with a remote processor 102 via a wired or wireless connection. For example, the processor 102 may form part of a computing device, such as a wearable device, a smart phone, a tablet computer, a laptop computer or a desktop computer, or may comprise a cloud-based processing facility.

The functions performed by the processor 102 described above may form part of a computer-implemented method, such as a computer-implemented method for measuring a concentration of a biomarker in sweat generated by a sweat gland of a subject. For example, such a computer-implemented method may be carried out using a processor and may comprise: operating a biomarker sensor to measure a first concentration of a biomarker in a first volume of sweat secreted from the sweat gland while a pressure application unit is not applying a pressure to the subject; operating a pressure application unit to apply a positive pressure to the subject at the location of the sweat gland; operating the biomarker sensor to measure a second concentration of the biomarker in a second volume of sweat secreted from the sweat gland while the pressure application unit is applying a positive pressure to the subject; and calculating, based on the determined first concentration and the measured second concentration, a third concentration of the biomarker, the third concentration relating to the concentration of the biomarker in the blood of the subject.

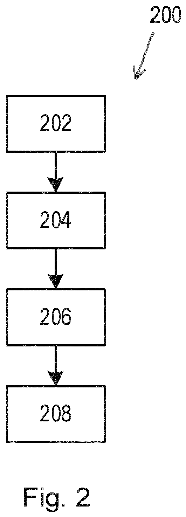

A more general method is discussed with reference to Fig. 2. Fig. 2 is a flowchart of an example of a method 200 for measuring a concentration of a biomarker in sweat generated by a subject. The method 200 comprises, at step 202, determining a first concentration of the biomarker in sweat secreted from a sweat gland of the subject. In some embodiments, the first concentration of the biomarker may be determined by measuring the first concentration, for example using the biomarker sensor 104 discussed herein. In other embodiments, determining the first concentration may comprise obtaining or retrieving the first concentration from a database or lookup table. For example, the database or lookup table may comprise concentrations of various biomarkers for various subjects or groups/populations of subjects, such as demographic groups of subjects. Various biomarkers may be detectable in sweat, and the particular biomarker whose concentration is to be measured varies from case to case. In some embodiments, a concentration of lactate may be measured in the sweat, for example when a positive external pressure is applied. When a negative external pressure is applied, a concentration of various ions (e.g. sodium ions, chlorine ions and potassium ions) may be measured in the sweat. In other embodiments, the concentration of other biomarkers may be measured or determined. As noted above, while no external pressure is being applied to the subject (e.g. to the tissue surrounding the sweat glands from which the measured sweat originates), the concentration of the biomarker measured or determined in the sweat results from a combination of the concentration of the biomarker generated by the sweat glands and the concentration of the biomarker resulting from blood moving into the interstitial fluid and into the sweat gland from the capillaries by capillary exchange and/or filtration.

At step 204, the method 200 comprises applying an external positive pressure to the subject at the location of the sweat gland. In some embodiments, the external positive pressure may be applied for a defined duration. The external positive pressure may be applied using the pressure application unit 106 discussed herein. In some embodiments, applying an external positive pressure may comprise exerting a force onto skin of the subject at the location of the sweat gland, so as to increase a pressure (e.g. a hydrostatic pressure) in interstitial fluid around the sweat gland. For example, an external positive pressure may be applied to the subject using a cuff (e.g. an arm cuff, a wrist cuff or a finger cuff) configured to inflate while positioned around the arm, wrist or finger of the subject, and apply a pressure to tissues within the subject. In some embodiments, after a positive pressure has been applied, a negative pressure may be applied. Applying a negative pressure to the subject at the location of the sweat gland may reduce a pressure (e.g. a hydrostatic pressure) in interstitial fluid around the sweat gland, and may be used in addition to a positive pressure as discussed below.

The method 200 comprises, at step 206, measuring, while the external positive pressure is being applied, a second concentration of the biomarker in sweat secreted from the sweat gland of the subject. The positive pressure may be applied to the subject for a defined duration which is intended to be sufficient to allow blood flow within the subject's body to adapt to the increased pressure. This defined duration may for example be at least 1 minute, preferably at least 5 minutes, and more preferably at least 8 minutes, e.g. in the order of 10 minutes. As noted above, the application of an external positive pressure increases the hydrostatic pressure in the interstitial fluid, thereby reducing the transport of fluid from capillaries into the interstitial fluid by filtration. Once the application of external positive pressure has begun, the change in the fluid transport within the subject's body is given time to adapt (i.e. during the defined duration), and the second concentration of the biomarker is then measured. As noted above, during the application of external positive pressure, the concentration of the biomarker measured in the sweat result mainly from the generation of the biomarker by the sweat glands themselves (e.g. as a metabolite due to the metabolism of the sweat glands).

At step 208, the method 200 comprises calculating, based on the determined first concentration and the measured second concentration, a third concentration of the biomarker, the third concentration relating to the concentration of the biomarker in the blood of the subject. As discussed above, in some embodiments, calculating the third concentration of the biomarker may comprise subtracting the measured second concentration from the determined first concentration.

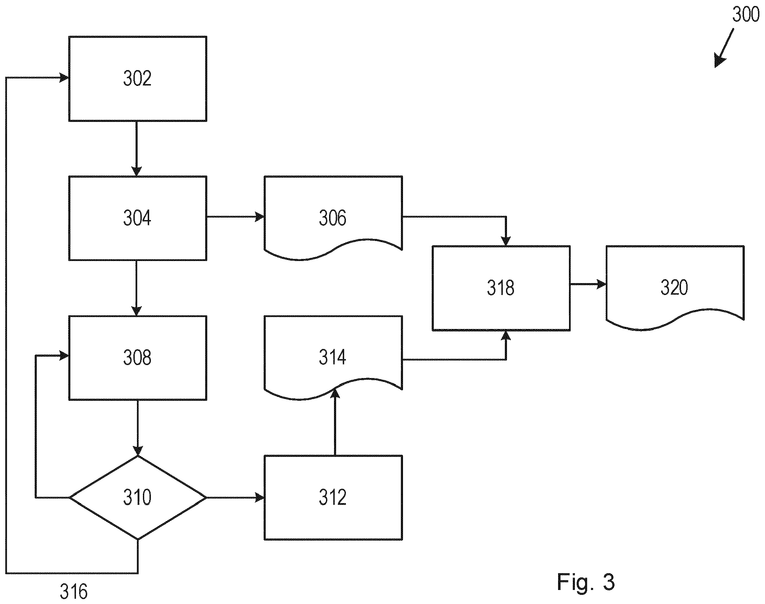

Fig. 3 is a flowchart of a further example of a method 300 of measuring a concentration of a biomarker in sweat generated by a subject. The method 300 begins at block 302, where an external pressure to be applied to the subject (e.g. by the pressure application unit 106) is set to zero. At block 304, a first concentration of the biomarker in sweat generated by sweat glands of the subject is measured. The block 304 may comprise or be similar to the step 202 of the method 200. An output of the measurement performed at block 304 is a signal 306 indicative of a total concentration of the biomarker, resulting from generation of the biomarker by the sweat glands and from transport of the biomarker from the capillary bed into the sweat glands. At block 308, an external positive pressure is applied to the subject. In some embodiments, the external positive pressure may be applied by the pressure application unit 106, such as a cuff. The positive pressure to be applied is set to a defined pressure which may be equal to or greater than the hydrostatic blood pressure of the subject. In some embodiments, a blood pressure measurement device may be used to apply the external positive pressure to the subject, and this may be used to measure the hydrostatic blood pressure of the subject. Setting the pressure to be applied to the subject at block 308 may be based on the blood pressure measured using the blood pressure measurement device. The block 308 may comprise or be similar to the step 204 of the method 200.

The method 300 continues to block 310, at which a timer or counter is started. The timer or counter is used to time the duration that the pressure is applied to the subject. If the expired time is less than a defined duration, then the method 300 returns to block 308 and application of the external positive pressure is continued. While the external positive pressure is being applied to the subject and, in some embodiments, after the positive pressure has been applied to the subject for a defined threshold amount of time, the method 300 proceeds to block 312, at which a second concentration of the biomarker in sweat generated by the sweat glands is measured. The block 312 may comprise or be similar to the step 206 of the method 200. An output of the measurement performed at block 312 is a signal 314 indicative of a concentration of the biomarker resulting just from generation of the biomarker by the sweat glands (i.e. not including the contribution from transport of the biomarker from the capillary bed into the sweat glands). Once the second biomarker concentration measurement has been made at block 312, or after expiry of a further defined threshold time measured by the timer or counter, the method may return (via 316) to block 302 where the external positive pressure is released and set to zero.

At block 318 of the method 300, a determination is made of third concentration of the biomarker that relates to the concentration of the biomarker in the blood of the subject. Specifically, the determined third concentration of the biomarker relates to the concentration resulting just from the transport of biomarker in fluid from the capillary bed into the sweat glands. Thus, an output of the measurement performed at block 318 is a signal 320 indicative of the concentration of the biomarker resulting from the capillary bed. The block 318 may comprise or be similar to the step 208 of the method 200.

For some biomarkers, such as lactate, application of an external positive pressure on the subject not only reduces the influx of the biomarker from the capillary bed into the sweat glands (and therefore into the sweat) but, over time, may also reduce the influx of oxygen into cells of the sweat glands. This oxygen deprivation can bring about a change in the sweat gland cells, whereby the cells eventually switch to an anaerobic metabolism process which, in turn leads to an increased concentration some biomarkers (e.g. lactate) in sweat after a prolonged duration. Consequently, the measured concentration of some biomarkers may even increase due to the switch to anaerobic metabolism, rather than decreasing due to the application of external pressure.

To take account of and compensate for this effect, the method may, in some embodiments, make use of calibration values that can be generated *a priori*. For example, a curve may be rendered showing the concentration of the biomarker (e.g. lactate) in sweat as a function of average sweat rate per gland). The calibration values (e.g. the curve) may be used to estimate the rate of change of lactate concentration as function of the sweat rate.

In some embodiments, such a calibration may be performed in respect of the subject whose sweat is being analysed. For example, before external pressure is applied to subject, the sweat rate of the subject may be measured along with the first concentration of the biomarker (e.g. lactate). The sweat rate of the subject may be measured using a sweat rate measurement unit, which may measure an amount of sweat generated by a sweat gland or by a group of sweat glands in a defined time. When the external positive pressure is applied, the sweat rate may be measured again, along with the second concentration of the biomarker. From the concentration of the biomarker measured without the application of external pressure, a rate of change of the sweat rate may be applied, as noted above, and this may provide a good estimate of the expected concentration of the biomarker resulting from the capillary filtration (i.e. from transport from the blood to the sweat glands).

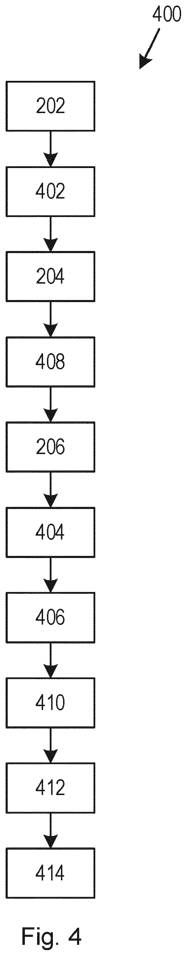

Fig. 4 is a flowchart of a further example of a method 400 for measuring a concentration of a biomarker in sweat generated by subject. The method 400 may comprise steps of the method 200 discussed above. The calibration steps discussed above may be performed as part of the method 400. In some embodiments, the method 400 may comprise, at step 402, measuring, prior to applying the external pressure to the subject (step 204), a first sweat rate of the subject. At step 404, the method 400 may comprise measuring, after expiry of a defined duration, a second sweat rate of the subject. The defined duration may be selected based on the time taken for a pressure equilibrium within the subject to be reached and it may for example be at least 1 minute, preferably at least 5 minutes, and more preferably at least 8 minutes, e.g. in the order of 10 minutes. For example, the second sweat rate may be measured while the external positive pressure is being applied, after a defined duration to allow the internal pressure of the subject's body to adapt to the external pressure being applied. Calculating the third concentration of the biomarker (step 208) may be further based on the measured first sweat rate and the measured second sweat rate.

Once the external positive pressure has been removed, the capillary function normalises, leading to the excretion of some biomarkers such as lactate by the sweat glands. These different states (e.g. zero pressure, pressure build-up and pressure release) may lead to different rates of biomarker measurement, particularly lactate. These production rates may, in some embodiments, be monitored in order to determine the minimum value of biomarker (e.g. lactate) concentration. In some embodiments, measurements of oxygen saturation (i.e. SpO2) may be made in the tissue in order to monitor the anaerobic state. This may be achieved either by measuring the oxygenation of the tissue with known optical methods or by measuring SpO2 downstream (for example in the fingers) by known methods.

In one embodiment, applying the external pressure (step 204) comprises applying a positive pressure to a surface of the subject at the location of the sweat gland. In this embodiment, the method 400 may further comprise, at step 406, applying, after measuring the second concentration of the biomarker (step 206), a negative pressure to the subject at the location of the sweat gland. In some embodiments, as discussed below, it may be possible to apply a negative pressure to the subject using the same device or unit (e.g. the pressure application unit 106) used to apply a positive pressure to the subject. In this way, the benefits of applying both a positive pressure and a negative pressure can be realised in rapid succession. The application of a negative pressure can be used to determine a change in the concentration of ions in the sweat of the subject, including sodium ions, chlorine ions and potassium ions, for example, which corresponds to the concentration of ions in the subject's blood. Thus, by applying a negative pressure, it may be possible to determine a concentration of ions in the blood in addition to the concentration of other biomarkers, such as lactate.

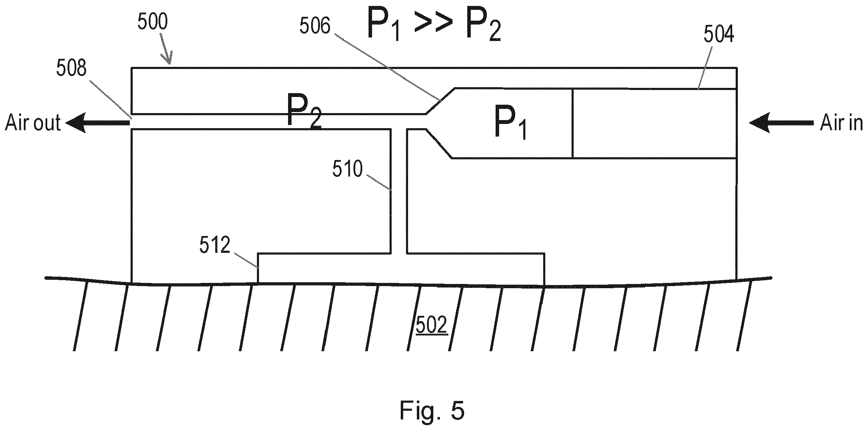

Several different techniques may be used to apply a negative pressure to the skin of a subject. Fig. 5 is a schematic illustration of an example of one technique by which a negative pressure may be applied. The technique shown in Fig. 5 utilises the Venturi effect, or the Bernoulli principle. In the example shown in Fig. 5, a device 500, which may include a sweat sensor (not shown) is positioned on skin of a subject 502. The device 500 includes a compressor or fan 504 to blow air or fluid (e.g. old sweat) at high speed (e.g. between 10 and 100 m/s) through a converging channel 506 towards an outlet 508. The channel 506 is connected to a further channel 510, leading to a chamber 512 formed between the device and the skin of the subject 502. As the air or fluid is pumped through the converging channel 506, an under-pressure (e.g. a partial vacuum) is formed in the chamber 512. The formation of this under-pressure is governed by Bernoulli's equation:

$$P_0 = P_1 + 0.5\rho_1 U_1^2 = P_2 + 0.5\rho_2 U_2^2 \qquad\qquad [2]$$

where $P_0$ is the total pressure of a system, $P_1$ is the pressure at a first position in the system, $P_2$ is the pressure at a second position in the system, $\rho_1$ is the fluid density at the first position, $\rho_2$ is the is the fluid density at the second position, $U_1$ is the velocity of the air or fluid at the first position in the system, and $U_2$ is the velocity of the air or fluid at the second position in the system.

Assuming incompressible flow implies $\rho_1 = \rho_2 = \rho$, and given $U_1 \approx 0$, this yields:

$$P_1 = P_2 + 0.5\rho U_2^2 \qquad\qquad [3]$$

where $P_2 \ll P_1$. Regions having the pressures $P_1$ and $P_2$ are shown in Fig. 5.

The compressor or fan can be activated when sweat sampling is required. However, as sweat is produced and/or as a seal between the device 500 and the skin of the subject 502 begins to leak, the under-pressure will slowly reduce. If there is a requirement to maintain the under-pressure for a longer period of time, it may be necessary to re-activate the compressor or fan.

In other embodiments, an active pump may be used to create a reduced pressure, or used/wasted sweat may be used to drive a turbo motor in order to draw more sweat into the device.

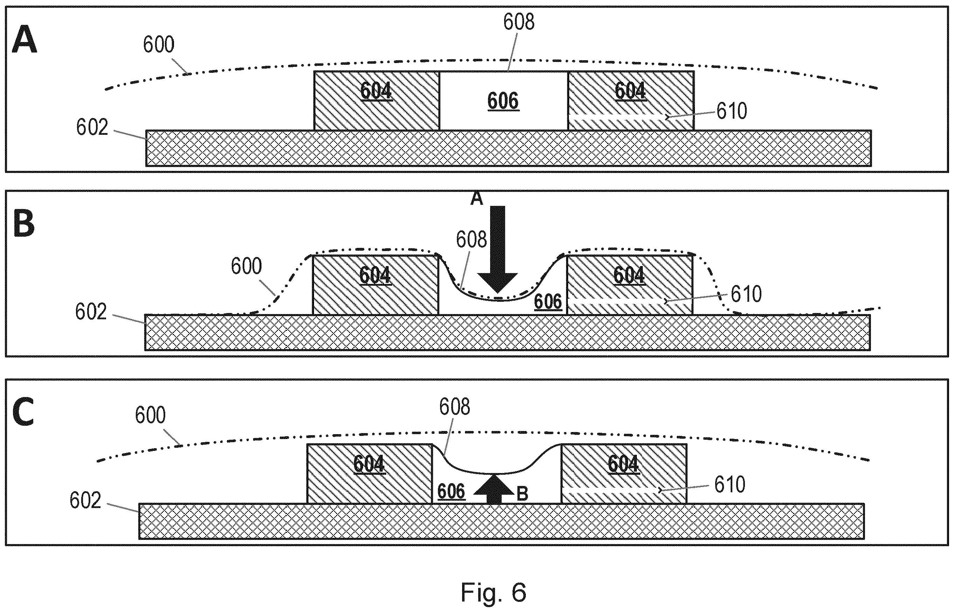

In an alternative embodiment, a device used for creating a positive pressure may also be used for creating a negative pressure. Fig. 6 is a schematic cross-sectional illustration of an arrangement in which a device 600 capable of creating a positive pressure is used to generate a negative pressure, or under-pressure. In this example, the device 600 comprises a cuff of a device used for measuring a subject's blood pressure. A substrate 604 is positioned between the cuff 600 and the subject's skin 602, such that the walls of the substrate form sidewalls of an enclosed chamber 606. For example, the substrate 604 may comprise a ring-shaped or annular structure. A flexible membrane 608 positioned between opposing sides of the substrate 604 forms a top wall of the chamber 606, and the skin of the subject 602 forms the bottom wall of the chamber. In some examples, the substrate 604 may comprise part of a wearable device or patch that includes a sweat sensor. A vent 610 is formed through the substrate 604 and is configured to allow the passage of fluid from the chamber 606 to the ambient air surrounding the substrate. A one-way valve may be provided to allow air out of the device through the vent 610, but preventing the flow of air into the device through the vent. In Fig. 6A, the cuff 600 is not inflated, so it is not applying a positive pressure to skin of the subject 602. Fig. 6B shows the arrangement when the cuff 600 is inflated, so as to apply a positive pressure to the subject 602. Inflation of the cuff 600 urges the flexible membrane downwards towards the skin of the subject 602, forcing air within the chamber 606 out through the vent 610, and generating a positive, downward pressure on the subject, as indicated by the arrow A. Fig. 6C shows the arrangement when the cuff 600 has deflated, such that the cuff no longer applies a positive pressure on the subject 602. Since air is unable to return through the vent 610 into the chamber 606, when the flexible membrane 608 tries to return to its original form (as shown in Fig. 6A), it creates a negative pressure (i.e. an under-pressure) within the cavity, as indicated by the arrow B. Thus, a standard "off-the-shelf' blood pressure cuff can be used to generate both a positive pressure and a negative pressure.

The application of a negative pressure to the subject can be particularly advantageous when measuring the concentration of particular biomarkers. The creation of a negative pressure causes ions in fluid to be forced out the subject's bloodstream (i.e. capillaries) into the interstitial fluid, and also into the sweat glands. Thus, the sweat glands have an increased concentration of ions, but it can be assumed that, due to the negative pressure, these are from the blood. Since the concentration of ions in the interstitial fluid is high, the reabsorption of the ions from the sweat glands and from the sweat ducts, that connect the glands to the surface of skin, into the interstitial fluid is greatly reduced. Therefore, the concentration of the ions (i.e. the biomarker) in sweat measured while a negative pressure is applied is approximately equal to the concentration of the biomarker in the blood of the subject.

Due to the passive transport of some biomarkers from blood to the interstitial fluid (and from the interstitial fluid to the sweat glands) there may be a lag in time between the biomarker concentration in blood corresponding to the biomarker concentration interstitial fluid. One example of where such a lag is evident is when measuring the concentration of glucose in interstitial fluid.

By applying a negative pressure to the subject, the hydrostatic pressure in the interstitial fluid ($P_i$) is reduced. If the hydrostatic blood pressure ($P_c$) is greater than the hydrostatic pressure in the interstitial fluid (i.e. if $P_c > P_i$), then there will be an excess of fluid flow from the capillary bed into the interstitial fluid. Consequently, the lag time will decrease until an equilibrium is reached between the hydrostatic pressure is Pi and $P_c$. During this phase, the lag time between the concentration of the biomarker in blood and the concentration of the biomarker in interstitial fluid is decreased.

In some embodiments, it may be desirable to apply an external pressure to the subject in order to reach an equilibrium between the hydrostatic pressure in blood ($P_c$) and the hydrostatic pressure in the interstitial fluid ($P_i$). For example, when it is desirable to control tissue perfusion (i.e. to create a situation where $P_i > P_c$) for a relatively long period of time (e.g. in the order of minutes), the vital transport of oxygen, carbon dioxide and nutrition may be hampered, potentially leading to tissue damage. By applying an external pressure equal to that of the hydrostatic (blood) pressure, such that Pi = $P_c$), filtration (i.e. the filtering of fluid containing biomarkers out of the capillaries) is reduced, but the diffusion of gases and, to some extent, nutrition and waste products is not hampered.

The hydrostatic blood pressure, $P_c$, can be measured using standard blood pressure measurements, for example using a blood pressure measurement cuff or from photoplethysmography (PPG) measurements. In both cases, the hydrostatic pressure of the interstitial fluid, $P_i$, is adapted to be the same as the measured hydrostatic blood pressure.

Referring again to Fig. 4, the method 400 may comprise, at step 408, measuring a blood pressure of the subject. The blood pressure may be measured prior to applying the external positive pressure or while the external positive pressure is being applied. In some embodiments, applying the external pressure (step 206) may comprise applying a positive pressure to equalise a pressure of interstitial fluid ($P_i$) around the sweat gland with the measured blood pressure ($P_c$). Thus, the external positive pressure applied to the subject may be adapted based on the measured blood pressure.

Filtration from capillaries is also driven by the oncotic pressure, as shown in equation [1] above. A normal oncotic pressure (e.g. for a healthy person) in plasma (referred to as $n_c$) is around 25 mmHg and a normal oncotic pressure of interstitial fluid (referred to as $n_i$) is around 3 mmHg. The main contributors to oncotic pressure in plasma are plasma proteins (mainly albumin). Since only $n_i$ contributes to the hydrostatic blood pressure $P_c$, it is a relatively small component and may, in some embodiments, be either added to the external positive pressure applied or, in other embodiments, it may be assumed that the effect is negligible.

In some embodiments, it may be advantageous to measure the concentration of a biomarker repeatedly over a period of time. In some examples, biomarker concentration measurement may be taken repeatedly during the application of the external positive pressure, for example while the pressure applied by the pressure application unit 106 is increasing and/or decreasing.

Measurements of biomarker concentrations taken during a ramped inflation and/or a ramped deflation of the pressure application unit (e.g. a cuff) over a period of time may be used to generate a calibration curve for the biomarker of interest. Thus, in some embodiments, the method 400 may comprise, at step 410, measuring, during the application of the external pressure (step 204), a plurality of concentrations of the biomarker. In such cases, calculating the third concentration (step 208) may be further based on the plurality of measurements. For example, a calibration curve may be generated based on the measured concentrations of the biomarker over time, for example as the applied pressure increases.

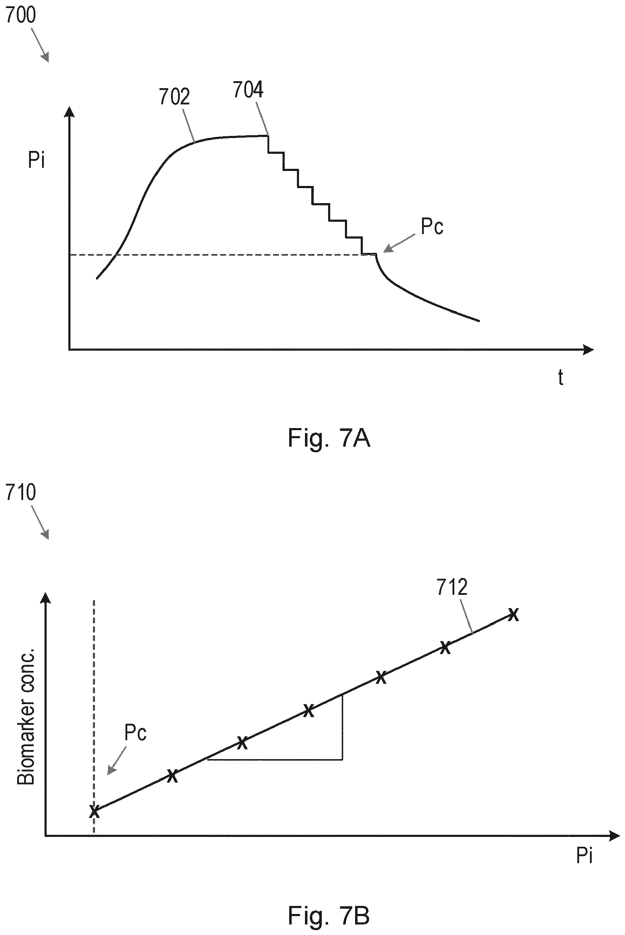

In some embodiments, pressure ramping may be performed in a manner similar to that used when taking blood pressure measurements using a blood pressure measurement cuff. The applied pressure $P_i$ may be increased to be equal to $P_c$, and then decreased in discrete steps until $P_i = P_c$ (i.e. around 35 mmHg). Fig. 7 is an illustration of two graphs relating to this embodiment. Fig. 7A is a graph 700 showing how the hydrostatic pressure of interstitial fluid, $P_i$, varies (line 702) as a function of time, as an external pressure is applied until $P_i$ reaches a maximum 704 and is then reduced in discrete steps. At each step, biomarker concentration measurements are taken over a period of time (e.g. in the order of minutes to tens of minutes) to enable a calibration curve between $P_i$ and the biomarker to be determined. Fig. 7B is a graph 710 showing how the concentration of the biomarker varies as a function of Pi, and the curve 712 plotted in this graph may be used as the calibration curve.

It may be intended that the step interval is long enough to permit changes in perfusion to influence the biomarker concentration measurements in sweat, but short enough to minimize the influence of changes in metabolism or sweat rate. Such a calibration step may be performed intermittently (e.g. daily, weekly or monthly) to account for changes in patient metabolism over time which may be influenced by medication administration, disease progression and fitness. Thus, the calibration may be considered to be a re-calibration.

Thus, with reference again to Fig. 4, the step of applying the external positive pressure (step 204) may comprise, after measuring the second concentration of the biomarker, decreasing the applied pressure in regular steps, and measuring, at each step, a concentration of the biomarker in sweat secreted from the sweat gland of the subject. The applied positive pressure may be decreased (e.g. the magnitude of the applied pressure may be reduced) and may, in some examples, remain above air pressure (e.g. the ambient air pressure). In other examples, the applied pressure may be decreased such that the pressure reduces to below the air pressure, and becomes a "negative" pressure. Thus, the applied pressure may start as a positive pressure and decrease to become a negative pressure, or vice versa. The method 400 may further comprise, at step 412, generating a calibration curve 712 using the measured concentrations. Calculating the third concentration of the biomarker (step 208) may be further based on the generated calibration curve. In other words, the calibration may be applied when calculating the third concentration of the biomarker.

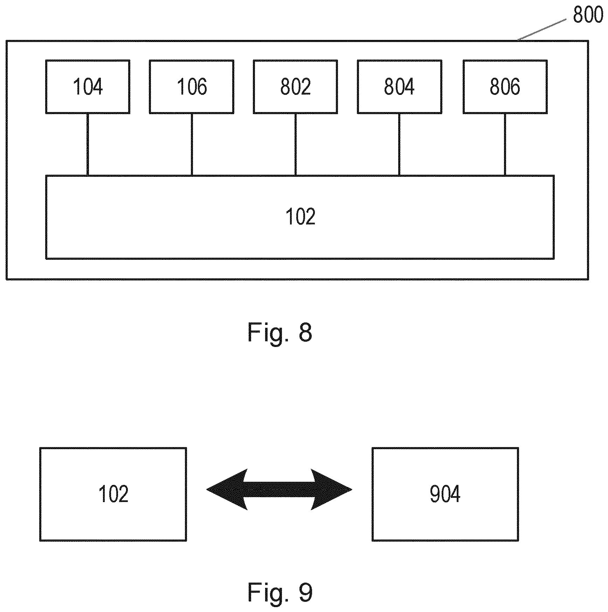

Fig. 8 is a schematic illustration of a further example of an apparatus 800 for measuring a concentration of a biomarker in sweat generated by a subject. The apparatus 800 may comprise, or be similar to, the apparatus 100 discussed above. Specifically, the apparatus 800 comprises a biomarker sensor 104, a pressure application unit 106 and a processor 102 in communication with the biomarker sensor and the pressure application unit. The processor 102 is configured to perform the functions discussed above in connection with Fig. 1.

As discussed above, the pressure application unit 106 may comprise at least one of: a unit configured to apply a positive pressure on the subject; and a unit configured to apply a negative pressure on the subject. In some embodiments, the pressure application unit 106 may be configured to apply both a positive pressure and a negative pressure, either successively or concurrently, using the techniques discussed herein.

In some embodiments, the biomarker sensor 104 may be configured to measure the concentration, in the volume of sweat, of a biomarker selected from a group comprising: lactate, sodium ions, potassium ions, chlorine ions, urea, ethanol, ammonium, glucose and cortisol. Multiple biomarker sensors may be included, each configured to measure the concentration of one or more biomarkers.

The apparatus 800 may, in some embodiments, further comprise a blood pressure measurement unit 802 for measuring a blood pressure of the subject. For example, the blood pressure measuring unit 802 may be configured to measure a hydrostatic blood pressure ($P_c$) of the subject. The blood pressure measurement unit 802 may be in communication with the processor 102. The processor 102 may be configured to operate the pressure application unit 106 to apply a positive pressure based on the measured blood pressure of the subject.

In some embodiments, the blood pressure measurement unit 802 may be configured to measure a capillary hydrostatic pressure ($P_c$) of the subject. The processor 102 may be configured to operate the pressure application unit 106 to apply a positive pressure to the subject such that a hydrostatic pressure in interstitial fluid ($P_i$) around the sweat

gland is increased to equal the measured capillary hydrostatic pressure ($P_c$). In other words, in some embodiments, the pressure application unit 106 may be operated to apply a positive pressure approximately equal to the blood pressure measured by the blood pressure measuring unit 802.

The apparatus 800 may, in some embodiments, further comprise a timer 804 in communication with the processor 102, and configured to measure at least one of: a duration of application of the applied pressure; and the timing of measurements of the biomarker concentrations. The timer 804 may comprise or be similar to the timer or counter referred to in block 310 of Fig. 3.

The apparatus 800 may further comprise a sweat rate measurement unit 806 configured to measure a sweat rate of the subject. In some embodiments, the sweat rate measurement unit 806, which may be in communication with the processor 102, may form part of another component of the apparatus 800. The processor 102 may be configured to control the sweat rate measurement unit 806 to measure a first sweat rate of the subject prior to applying the external positive pressure to the subject, and control the sweat rate measurement unit to measure a second sweat rate of the subject after expiry of a defined duration. The processor 102 may be configured to calculate the third concentration of the biomarker further based on the measured first sweat rate and the measured second sweat rate, as discussed above.

The processor 102 of the apparatus 800 may, in some embodiments, be configured to operate the biomarker sensor 104 to measure a plurality of concentrations of the biomarker in volumes of sweat secreted from the sweat gland while the pressure application unit 106 is applying a positive pressure to the subject. The processor 102 may be configured to calculate the third concentration further based on the plurality of biomarker concentration measurements, as discussed above.

Fig. 9 is a schematic illustration of an example of a processor 902 in communication with a computer-readable medium 904. The processor 902 may comprise or be similar to the processor 102 discussed above. According to some embodiments, a computer program product comprises a non-transitory computer-readable medium 904, the computer-readable medium having computer-readable code embodied therein, the computer-readable code being configured such that, on execution by a suitable computer or processor 102, 902, the computer or processor is caused to or determine a first concentration of a biomarker in a first volume of sweat secreted from a sweat gland of the subject while an external pressure is not being applied to the subject by a pressure application unit (e.g. the pressure application unit 106); operate the pressure application unit to apply a positive pressure to the subject at the location of the sweat gland; operate a biomarker sensor to measure a second concentration of the biomarker in a second volume of sweat secreted from the sweat gland while the pressure application unit is applying a positive pressure to the subject; and calculate, based on the determined first concentration and the measured second concentration, a third concentration of the biomarker, the third concentration relating to the concentration of the biomarker in the blood of the subject. In other embodiments, the computer-readable medium may comprise code configured such that, on execution, the computer or processor is further caused to perform one or more of the steps or functions discussed herein. In some embodiments, the computer or processor may be caused to determine the first concentration by operating a biomarker sensor (e.g. the biomarker sensor 104) to measure the first concentration while, in other embodiments, the computer or processor may be caused to determine the first concentration by obtaining the first concentration from a database or look up table.

The processor 102, 902 can comprise one or more processors, processing units, multicore processors or modules that are configured or programmed to control the apparatus 100, 800 in the manner described herein. In particular implementations, the processor 102, 902 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. Measures recited in mutually different dependent claims can be advantageously combined. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A method (200) for measuring a concentration of a biomarker in sweat generated by a subject, the method comprising:

   determining (202) a first concentration of the biomarker in sweat secreted from a sweat gland of the subject;
   applying (204) an external positive pressure to the subject at the location of the sweat gland;
   measuring (206), while the external positive pressure is being applied, a second concentration of the biomarker in sweat secreted from the sweat gland of the subject; and
   calculating (208), based on the determined first concentration and the measured second concentration, a third concentration of the biomarker, the third concentration relating to the concentration of the biomarker in the blood of the subject.

2. A method (200, 400) according to claim 1, further comprising:

   measuring (402), prior to applying the external positive pressure to the subject, a first sweat rate of the subject; and
   measuring (404), after expiry of a defined duration, a second sweat rate of the subject;
   wherein calculating the third concentration of the biomarker is further based on the measured first sweat rate and the measured second sweat rate.

3. A method (200, 400) according to claim 1 or claim 2, wherein applying an external positive pressure comprises exerting a force onto skin of the subject at the location of the sweat gland, so as to increase a pressure in interstitial fluid around the sweat gland.

4. A method (200, 400) according to any of the preceding claims, further comprising:
   applying (406), after measuring the second concentration of the biomarker, a negative pressure to the subject at the location of the sweat gland.

5. A method (200, 400) according to any of the preceding claims, further comprising:

   measuring (410), during the application of the external positive pressure, a plurality of concentrations of the biomarker;
   wherein calculating the third concentration is further based on the plurality of measurements.

6. A method (200, 400) according to any of the preceding claims, further comprising:

   measuring (408) a blood pressure of the subject;
   wherein applying the external positive pressure comprises applying a positive pressure to equalise a pressure of interstitial fluid around the sweat gland with the measured blood pressure.

7.  A method (200, 400) according to any of the preceding claims, wherein applying the external positive pressure comprises, after measuring the second concentration of the biomarker:

    decreasing the applied pressure in regular steps; and
    measuring, at each step, a concentration of the biomarker in sweat secreted from the sweat gland of the subject; wherein the method further comprises:

    generating (412) a calibration curve using the measured concentrations; and
    wherein calculating the third concentration of the biomarker is further based on the generated calibration curve.

8.  An apparatus (100, 800) for measuring a concentration of a biomarker in sweat generated by a subject, the apparatus comprising:

    a biomarker sensor (104) configured for receiving a volume of sweat secreted from a sweat gland of the subject and measuring a concentration of a biomarker in the volume of sweat;
    a pressure application unit (106) configured to apply a positive pressure to the subject at the location of the sweat gland; and
    a processor (102) in communication with the biomarker sensor and the pressure application unit, and configured to:

    determine a first concentration of the biomarker in a first volume of sweat secreted from the sweat gland while the pressure application unit is not applying a pressure to the subject;
    operate the pressure application unit to apply a positive pressure to the subject at the location of the sweat gland;
    operate the biomarker sensor to measure a second concentration of the biomarker in a second volume of sweat secreted from the sweat gland while the pressure application unit is applying a positive pressure to the subject; and
    calculate, based on the determined first concentration and the measured second concentration, a third concentration of the biomarker, the third concentration relating to the concentration of the biomarker in the blood of the subject.

9.  An apparatus (100, 800) according to claim 8, further comprising:

    a blood pressure measurement unit (802) for measuring a blood pressure of the subject;
    wherein the processor is configured to operate the pressure application unit to apply a positive pressure based on the measured blood pressure of the subject.

10. An apparatus (100, 800) according to claim 9, wherein the blood pressure measurement unit is configured to measure a capillary hydrostatic pressure of the subject; and
    wherein the processor is configured to operate the pressure application unit to apply a positive pressure to the subject such that a hydrostatic pressure in interstitial fluid around the sweat gland is increased to equal the measured capillary hydrostatic pressure.

11. An apparatus (100, 800) according to any to claims 8 to 10, further comprising:
    a timer (804) in communication with the processor, and configured to measure at least one of: a duration of application of the applied pressure; and the timing of measurements of the biomarker concentrations.

12. An apparatus (100, 800) according to any to claims 8 to 11, further comprising:

    a sweat rate measurement unit (806) configured to measure a sweat rate of the subject;
    wherein the processor is configured to:

    control the sweat rate measurement unit to measure a first sweat rate of the subject prior to applying the external positive pressure to the subject;
    control the sweat rate measurement unit to measure a second sweat rate of the subject after expiry of a defined duration; and
calculate the third concentration of the biomarker further based on the measured first sweat rate and the measured second sweat rate.

13. An apparatus (100, 800) according to any to claims 8 to 12, wherein the processor is configured to:

operate the biomarker sensor to measure a plurality of concentrations of the biomarker in volumes of sweat secreted from the sweat gland while the pressure application unit is applying a positive pressure to the subject; and
calculate the third concentration further based on the plurality of biomarker concentration measurements.

14. An apparatus (100, 800) according to any to claims 8 to 13, wherein the biomarker sensor is configured to measure the concentration, in the volume of sweat, of a biomarker selected from a group comprising: lactate, sodium ions, potassium ions, chlorine ions, urea, ethanol, ammonium, glucose and cortisol.

15. A computer program product comprising a non-transitory computer-readable medium (904), the computer-readable medium having computer-readable code embodied therein, the computer-readable code being configured such that, on execution by a suitable computer or processor, the computer or processor (902) is caused to carry out the method for measuring a concentration of a biomarker in sweat generated by a subject as defined in any of claims 1 to 7.

Fig. 1

200

202

204

206

208

Fig. 2

Fig. 3

EP 4 085 837 A1

400

```
┌─────────────┐
│     202     │
└─────────────┘
       │
       ▼
┌─────────────┐
│     402     │
└─────────────┘
       │
       ▼
┌─────────────┐
│     204     │
└─────────────┘
       │
       ▼
┌─────────────┐
│     408     │
└─────────────┘
       │
       ▼
┌─────────────┐
│     206     │
└─────────────┘
       │
       ▼
┌─────────────┐
│     404     │
└─────────────┘
       │
       ▼
┌─────────────┐
│     406     │
└─────────────┘
       │
       ▼
┌─────────────┐
│     410     │
└─────────────┘
       │
       ▼
┌─────────────┐
│     412     │
└─────────────┘
       │
       ▼
┌─────────────┐
│     414     │
└─────────────┘
```

Fig. 4

$P_1 >> P_2$

500

506

504

508

$P_2$

$P_1$

Air out

Air in

510

512

502

Fig. 5

EP 4 085 837 A1

19

Fig. 6

Fig. 7A

Fig. 7B

800

| 104 | 106 | 802 | 804 | 806 |

102

Fig. 8

| 102 | | 904 |

Fig. 9

Europäisches
Patentamt

European
Patent Office

Office européen
des brevets

## EUROPEAN SEARCH REPORT

Application Number

EP 21 17 2335

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/008581 A2 (FREELANCE CORP [US]; FINKELSHTEIN IRINA V [US] ET AL.) 20 January 2011 (2011-01-20) * paragraphs [0001], [0094], [0099]; claim 30 * | 1,3,8, 11,14,15 | INV. A61B5/145 |
| A | US 2007/027383 A1 (PEYSER THOMAS A [US] ET AL) 1 February 2007 (2007-02-01) * paragraphs [0060], [0099] * | 1-15 | |

TECHNICAL FIELDS
SEARCHED (IPC)

A61B

The present search report has been drawn up for all claims

| Place of search | Date of completion of the search | Examiner |
|---|---|---|
| The Hague | 26 October 2021 | Knüpling, Moritz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 2335

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2021

| Patent document cited in search report | Publication date | Patent family member(s) | | Publication date |
|---|---|---|---|---|
| WO 2011008581 A2 | 20-01-2011 | AU | 2010273741 A1 | 01-03-2012 |
| | | CA | 2778773 A1 | 20-01-2011 |
| | | EP | 2454587 A2 | 23-05-2012 |
| | | US | 2012165626 A1 | 28-06-2012 |
| | | WO | 2011008581 A2 | 20-01-2011 |
| US 2007027383 A1 | 01-02-2007 | AU | 2007258618 A1 | 21-12-2007 |
| | | CA | 2654980 A1 | 21-12-2007 |
| | | CN | 101489470 A | 22-07-2009 |
| | | EP | 2034880 A1 | 18-03-2009 |
| | | IS | 8770 A | 28-11-2008 |
| | | JP | 2009539549 A | 19-11-2009 |
| | | RU | 2008152759 A | 20-07-2010 |
| | | US | 2007027383 A1 | 01-02-2007 |
| | | US | 2007179371 A1 | 02-08-2007 |
| | | US | 2009270704 A1 | 29-10-2009 |
| | | WO | 2007146047 A1 | 21-12-2007 |

EPO FORM P0459

For more details about this annex : see Official Journal of the European Patent Office, No. 12/82